# EUROPEAN PATENT APPLICATION

(11) **EP 4 545 114 A1**
(43) Date of publication of application: **30.04.2025**
(21) Application number: 24208394.7
(22) Date of filing: 23.10.2024
(51) Int. Cl.: A61M 1/34, A61M 1/36

(54) **THERAPEUTIC PLASMA EXCHANGE SYSTEM AND APHERESIS KIT FOR FLUSH AND DIVERT PRIME STAGE WHEN USING AN ADSORPTION COLUMN**

(30) Priority: 26.10.2023 US 202363593477 P; 18.10.2024 US 202418920829
(71) Applicant: Fenwal, Inc., Lake Zurich, IL 60047 (US)
(72) Inventor: NGUYEN, Lan T., Lake Zurich, 60047 (US); COULTAS, Gregory, Lake Zurich, 60047 (US)
(74) Representative: Maikowski & Ninnemann Patentanwälte Partnerschaft mbB

(57) **Abstract**

A flow circuit for a blood treatment system includes flow cassettes, a separation chamber fluidically connected to the cassettes, tubings fluidically connecting the cassettes and the separation chamber, the tubings including a plasma tubing including a first column connector, a Luer activated port, and a check valve. The flow circuit has a first configuration for a replacement fluid therapeutic plasma exchange procedure in which the first column connector has first and second mating parts connected to one another, and a second configuration for an adsorption therapeutic plasma exchange procedure in which the flow circuit further comprises an adsorption device having a second column connector and the adsorption device is connected in line with plasma tubing and the first and second mating parts of the first column connector are connected to corresponding first and second parts of the second column connector.

## Description

### Background

### Field of the Disclosure

The disclosure relates to blood treatment systems and methods. More particularly, the disclosure relates to systems and methods for flushing and divert prime stages in an apheresis kit configured for use in replacement fluid therapeutic plasma exchange and adsorption column therapeutic plasma exchange.

### Description of Related Art

Various blood processing systems now make it possible to collect particular blood constituents, rather than whole blood, from donors or patients. Typically, in such systems, whole blood is drawn from a donor, the particular blood component or constituent is removed and collected, and the remaining blood constituents are returned to the donor. By thus removing only particular constituents, potentially less time is needed for the donor's body to return to pre-donation levels, and donations can be made at more frequent intervals than when whole blood is collected. This increases the overall supply of blood constituents, such as plasma and platelets, made available for health care.

Whole blood is typically separated into its constituents through centrifugation. This requires that the whole blood be passed through a centrifuge after it is withdrawn from, and before it is returned to, the donor. To avoid contamination and possible infection of the donor, the blood is preferably contained within a sealed, sterile fluid flow system during the entire centrifugation process. Typical blood processing systems thus include a permanent, reusable centrifuge assembly containing the hardware (drive system, pumps, valve actuators, programmable controller, and the like) that spins and pumps the blood, and a disposable, sealed and sterile fluid processing assembly that is mounted in cooperation on the hardware. The centrifuge assembly engages and spins a disposable centrifuge chamber in the fluid processing assembly during a blood separation step. The blood, however, makes actual contact only with the fluid processing assembly, which assembly is used only once and then discarded.

As the whole blood is spun by the centrifuge, the heavier (greater specific gravity) components, such as red blood cells, move radially outwardly away from the center of rotation toward the outer or "high-G" wall of a separation chamber included as part of the fluid processing assembly. The lighter (lower specific gravity) components, such as plasma, migrate toward the inner or "low-G" wall of the separation chamber. Various ones of these components can be selectively removed from the whole blood by forming appropriately located channeling seals and outlet ports in the separation chamber of the fluid processing assembly.

One procedure using such a blood processing system is known as therapeutic plasma exchange or TPE. One application of TPE involves removing whole blood from a patient, separating plasma from cellular blood components, collecting the plasma, and returning the cellular blood components and a replacement fluid for the plasma to the patient.

In another application of TPE, rather than replacing a patient's plasma with a different fluid, the patient's own plasma may be treated and returned after separation. This may be most efficiently achieved by pairing the blood separation system with a secondary processing device, such as an adsorption device or column. The adsorption device will remove undesirable substances from the plasma by immuno-adsorption. The exact substances removed depend upon the needs of the patient. For example, the substances removed from the plasma by the adsorption device may include low-density lipoproteins and Lipoprotein(a) for patients suffering from severe hypercholesterolemia. In another example, pathogenic antibodies may be removed from the plasma, for patients suffering from autoimmune diseases and organ transplant rejection, or as a pre-treatment before transplantation. In yet another example, fibrinogen, fibrin, and/or C-reactive protein may be removed from the plasma, for treating microcirculation disorders and ischemic tissue damage. Exemplary adsorption devices include the TheraSorb^{®} line of products from Miltenyi Biotec GmbH Corporation of Bergisch Gladbach, Germany. Other examples of adsorption devices suitable for removing undesirable substances from plasma are described in greater detail in U.S. Patent No. 6,569,112 to Strahilevitz, which is incorporated herein by reference.

Currently, different TPE applications (i.e., replacement fluid for plasma and plasma adsorption) require the use of separate, differently configured, disposable, sealed sterile fluid processing assemblies. That is, a disposable fluid processing assembly designed for the replacement fluid TPE application above is not suitable for use in the plasma adsorption TPE application. For example, if a disposable processing assembly for the replacement fluid TPE application were used in the plasma adsorption TPE application by connecting an adsorption column to the fluid processing assembly, separated red cells may backflow to the adsorption column resulting in unintended and undesirable post-separation mixing of separated red blood cells and plasma.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a blood treatment system includes a reusable separation device having a plurality of cassette holders and a centrifuge, and a flow circuit having a plurality of cassettes. Each cassette is configured to be arranged on a corresponding cassette holder. The flow circuit further includes a separation chamber fluidically connected to the plurality of cassettes and configured to be arranged on the centrifuge, and a plurality of tubings fluidically connecting the plurality of cassettes and the separation chamber. The plurality of tubings include a plasma tubing having a first column connector, a Luer activated port, and a check valve. The flow circuit has a first configuration for a replacement fluid therapeutic plasma exchange procedure in which the first column connector has first and second mating parts connected to one another, and a second configuration for an adsorption therapeutic plasma exchange procedure in which the flow circuit further includes an adsorption device having a second column connector. The adsorption device is connected in line with plasma tubing and the first and second mating parts of the first column connector are connected to corresponding first and second parts of the second column connector.

In another aspect, a flow circuit for a blood treatment system, includes a plurality of cassettes, a separation chamber fluidically connected to the plurality of cassettes and a plurality of tubings fluidically connecting the plurality of cassettes and the separation chamber. The plurality of tubings include a plasma tubing having a first column connector, a Luer activated port, and a check valve. The flow circuit has a first configuration for a replacement fluid therapeutic plasma exchange procedure in which the first column connector has first and second mating parts connected to one another, and a second configuration for an adsorption therapeutic plasma exchange procedure in which the flow circuit further includes an adsorption device having a second column connector. The adsorption device is connected in line with the plasma tubing and the first and second mating parts of the first column connector are connected to corresponding first and second parts of the second column connector.

### Brief Description of the Drawings

Fig. 1 is a perspective view of an exemplary blood separation system that may be used in combination with disposable flow circuit, in accordance with an aspect of the present disclosure;
Fig. 2 is a diagrammatic view of an exemplary disposable flow circuit that may be used in combination with the centrifuge system of Fig. 1, arranged in a first configuration;
Fig. 3 is a first view of a fluid processing cassette of the flow circuit of Fig. 2;
Fig. 4 is a second view of the fluid processing cassette of Fig. 3;
Fig. 5 is a perspective view of a cassette holder of the separation device of Fig. 1;
Fig. 6 is a side elevational view, with portions broken away and in section, of the separation system of Fig. 1;
Fig. 7 is a diagrammatic view showing an example of the flow circuit of Fig. 2 in a second configuration;
Fig. 8 is a diagrammatic view showing an example of the flow circuit of Fig. 2 in a third configuration;
Fig. 9 is a diagrammatic view showing an example of the flow circuit of Fig. 2 in a fourth configuration; and
Fig. 10 is a diagrammatic view showing the flow circuit of Fig. 2 in a fifth configuration.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

Blood treatment systems according to the present disclosure include a reusable separation device and a disposable flow circuit configured to be connected to the blood separation device. The separation device is configured to interact with the flow circuit to direct flow of blood, blood components, or other fluids through the flow circuit in a predetermined manner to perform a therapeutic plasma exchange (TPE) procedure.

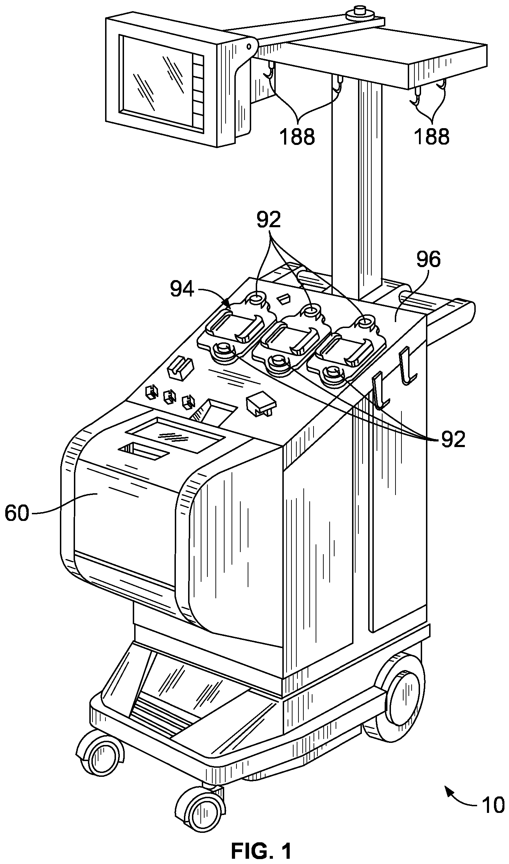

In the present examples, and with general reference to Figs. 1-10, the TPE procedure may be a replacement fluid procedure ("Replacement Fluid TPE") or an adsorption procedure ("Columns TPE"). In Replacement Fluid TPE, blood plasma is separated from a patient's blood and a replacement fluid is delivered to maintain the desired fluid balance. In this manner, the concentration of a pathologic substance in the patient's blood may be reduced. In Columns TPE, separated plasma is passed through an adsorption column which is configured to filter plasma before returning the plasma to the patient.

In the present examples, the flow circuit may be provided in a first configuration for a Replacement Fluid TPE procedure and may be reconfigured in at least a second configuration for a Columns TPE procedure. After configuring the flow circuit for a Columns TPE procedure, the present blood treatment system may be configured to perform a flush step to flush at least the adsorption device and a divert prime step to prime the flow circuit.

Referring generally to Figs. 1-3, the blood treatment system according to the present embodiments includes a reusable separation device 10 and disposable flow circuit 12 configured to be mounted on, and removed from, the separation device 10. The separation device 10 may be provided as a durable centrifuge system 10 that may be employed in blood treatment systems according to the present disclosure. The centrifuge system 10 may be provided according to known design, such as the system currently marketed as the AMICUS^{®} separator by Fenwal, Inc. of Lake Zurich, Illinois, as described in greater detail in U.S. Patent No. 5,868,696, which is hereby incorporated by reference in its entirety. The centrifuge system 10 can be used for processing various fluids but is particularly well suited for processing whole blood and other suspensions of biological cellular materials. While fluid treatment principles will be described herein with reference to one particular system, it should be understood that these principles may be employed with other blood separation devices without departing from the scope of the present disclosure. Further, it should also be appreciated that the blood treatment systems according to the present disclosure are not limited to blood separation by centrifugation, as the principles described herein may also be employed with blood separation devices which separate blood by other means, such as by a "spinning membrane" of the type described in U.S. Patent No. 5,194,145 to Schoendorfer, which is hereby incorporated herein by reference.

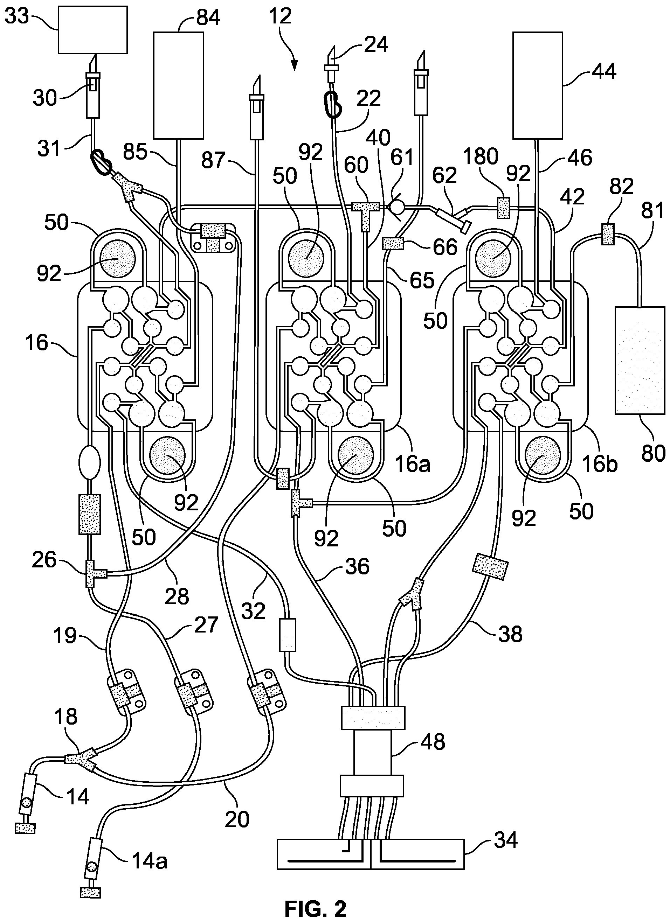

Fig. 2 illustrates an example of the disposable flow circuit 12 that may be used in combination with the centrifuge system 10 of Fig. 1, according to the present disclosure. The flow circuit 12 may be an apheresis kit. The illustrated flow circuit 12 includes, for example, a number of different tubes (also referred to as "tubing" or "tube segments"), left, middle and right cassettes 16, 16a, 16b fluidically connected to at least some of the tubes, an umbilicus 48 and a separation chamber 34 fluidically connected to the tubes and cassettes 16-16b.

The illustrated flow circuit 12 is a "two needle" system, which includes a pair of blood source access devices 14 and 14a (e.g., phlebotomy needles) for fluidly connecting a blood source (such as a patient or a container with previously collected blood) with the flow circuit 12. The blood source access devices 14 and 14a are connected by tubing to the left cassette 16, which is described further below. One of the blood source access devices 14 is used to draw blood from the blood source into the flow circuit 12 and is connected to the left cassette 16 by a first y-connector 18 and tubing 19. The other leg of the first y-connector 18 is connected to tubing 20 which leads to the middle cassette 16a. The tubing 20 is connected, through the middle cassette 16a, to additional tubing 22 having a container access device 24 (e.g., a sharpened cannula or spike connector) for accessing the interior of an anticoagulant container (not illustrated). During a blood treatment procedure, anticoagulant from the anticoagulant container may be received in the tubing 22, middle cassette 16a and tubing 20 to be combined with whole blood at first y-connector 18 prior to entering the left cassette 16.

The other blood source access device 14a is used to deliver or return blood, a blood component, and/or some other replacement fluid to the blood source and is connected to the left cassette 16 by a second y-connector 26 and tubing 27. The other leg of the second y-connector 26 is connected to tubing 28 connected at its other end to a container access device 30. The container access device 30 may be associated with a fluid supply container 33 having an amount of fluid to be used for flushing and priming the flow circuit 12. The fluid of the fluid supply container 33, in one embodiment, is saline, which may be used for flushing and priming the flow circuit 12.

The left cassette 16 also includes tubing 32 which is connected to the blood separation chamber 34 of the flow circuit 12 for flowing anticoagulated blood thereto. The blood separation chamber 34 separates the blood into its constituent parts (as described in greater detail below) and returns the blood components to the flow circuit 12. In one embodiment, the whole blood may be separated into cellular components (e.g., red blood cells) and substantially cell-free plasma. The cellular components may be returned to the middle cassette 16a from the separation chamber 34 via tubing 36, while the substantially cell-free plasma is returned to a right cassette 16b from the blood separation chamber 34 via tubing 38. The cellular blood components may be pumped to the left cassette 16 via tubing 40, and then returned to the blood source via tubing 27 and blood source access device 14a. The plasma may be pumped back to the left cassette 16 via tubing 42 (also referred to herein as the "plasma tubing 42") for return to the blood source and/or it may be pumped into a collection container 44 via different tubing 46. The destination of the plasma (and the other fluids passing through the cassettes) depends upon the actuation of the various valves of the cassettes, as described in greater detail below. The various tubing connected to the blood separation chamber 34 are bundled in the umbilicus 48.

Additional tubing may be connected from one port of a cassette to another port of the same cassette, so as to form tubing loops 50 which are configured to interact with a fluid flow element or pump of the device 10 to flow fluid through the flow circuit 12, as described in greater detail below.

With further reference to Fig. 2, the tubing 40 extends from the middle cassette 16a and is fluidically connected to the plasma tubing 42, for example by a third y-connector 60. A check valve 61 is connected to the plasma tubing 42 upstream from the third y-connector 60. As described further below, the check valve 61 may prevent backflow of blood constituents from the left cassette 16 or middle cassette 16a through the plasma tubing 42. For example, the check valve 61 may prevent backflow of cellular blood components during the divert prime stage for a Columns TPE procedure.

A Luer activated port 62 is also connected to the plasma tubing 42 and is arranged upstream from the check valve 61. The Luer activated port 62 may be connected to different tubing segments to provide different configurations of the flow circuit 12 as described further below.

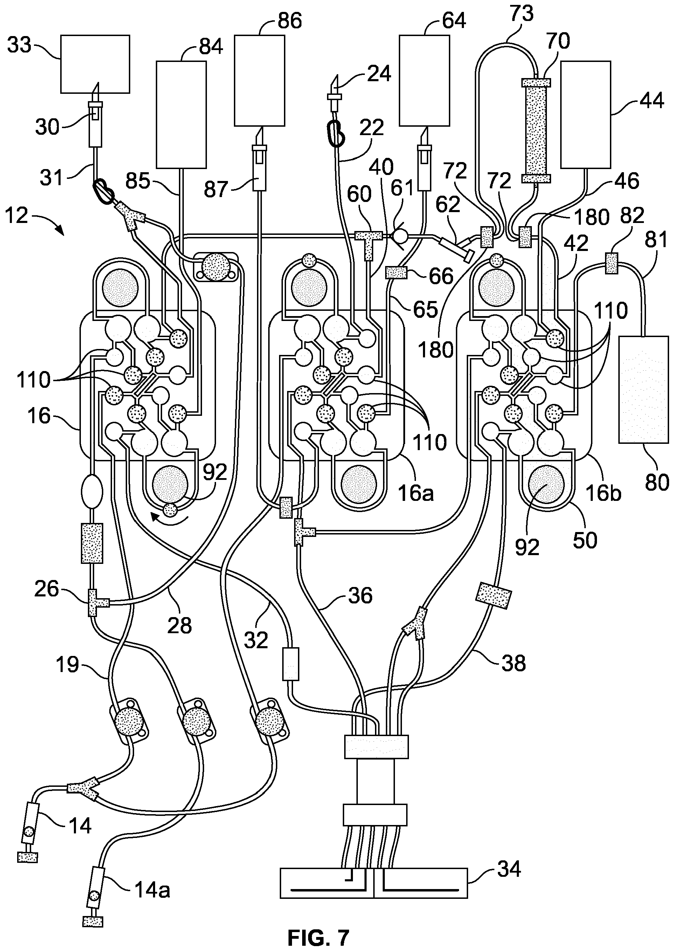

A first column connector 180 is also connected to the plasma tubing 42 to facilitate optional connection of an adsorption device 70 (see Figs. 7-10) used in a Columns TPE procedure (also referred to as an adsorption TPE procedure). In the illustrated example, the connector 180 comprises a Luer connector, which includes male and female mating parts which may be selectively connected and disconnected from one another, although a differently configured connector may also be employed without departing from the scope of the present disclosure. In the first, or initial, configuration of the flow circuit 12 the male and female mating parts of the first column connector 180 are connected to one another and allow for passage of fluid through the connector 180. In at least a second configuration of the flow circuit 12, the adsorption device 70 may be connected in line with the tubing 42, for example, by disconnecting the mating parts of the first column connector 180 from one another and connecting the mating parts of the first column connector 180 to corresponding mating parts of a second column connector 72 connected to the adsorption device 70. In this manner, the adsorption device 70 may be fluidically connected to the flow circuit 12 inline with the tubing 42. In such an example, the second column connector 72 may comprise a Luer connector having male and female parts configured for mating connection with the parts of the first column connector 180.

A second collection container 80 may be fluidically connected to the right cassette 16b, for example, by tubing 81 and a container connector 82. The container connector 82 may be Luer connector, which when connected, facilitates fluid flow into the second collection container 80, and when disconnected, substantially seals a free end of the connection tubing 81. The second collection container 80 may be a waste container configured to receive blood components and/or other fluids used in the flow circuit. In one example, the second collection container 80 may include multiple containers, and in a particular embodiment, may include two 2L capacity containers. The first collection container 44 may also be connected to the right cassette 16b via tubing 46, and may be provided as another waste container configured to receive a fluid from the right cassette 16b.

The adsorption device 70, as shown in Figs. 7-10, is configured to process separated plasma received from the blood separation chamber 34. Although the operation may differ depending on the device, an adsorption device 70 typically operates by providing a ligand or binding agent on a substrate. The substrate is packed into a housing (typically in the form of a column) and plasma is passed through the housing so as to come into contact with the substrate. The ligand binds to a particular substance (or substances) in the plasma, thereby removing the substance(s) from the plasma. The nature of the ligand depends on the substance(s) to be removed from the plasma. In known adsorption devices, antibodies and/or immune complexes may be removed from plasma using peptide-GAM or protein A as a ligand, while in other known devices, selected lipoproteins may be removed from plasma using dextran sulfate as a ligand. Other ligands may also be employed without departing from the scope of the present disclosure. Methods of selecting a ligand and preparing and using an adsorption device are described in U.S. Patent No. 5,277,701 to Christie et al., which is incorporated herein by reference.

Any of a variety of adsorption devices 70 may be employed in combination with the present blood separation system, including (but not limited to) the Globaffin^{™} and Immunosorba^{™} devices from Fresenius Medical Care Deutschland GmbH of Bad Homburg, Germany and the Liposorber^{®} device from Kaneka Corporation of Osaka, Japan.

The aforementioned blood separation chamber 34 may be of the type shown and described in US Pat. No. 10,413,652 assigned to FENWAL, INC. and incorporated herein by reference in its entirety. The blood separation chamber 34 may be suitable for separating whole blood therein, for example, for a TPE procedure. According to one example, the whole blood may be separated into cellular components (e.g., red blood cells) having a relatively high density and substantially cell-free plasma a relatively low density. The separated blood components may be removed from the separation chamber 34 in a known manner, for example, as described in previously referenced US Pat. No. 10,413,652

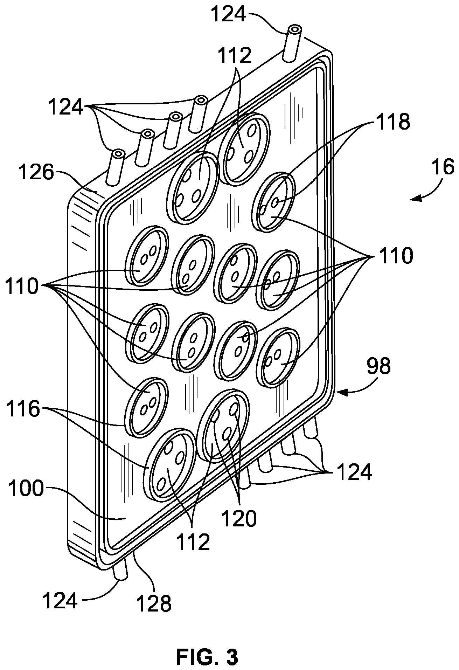

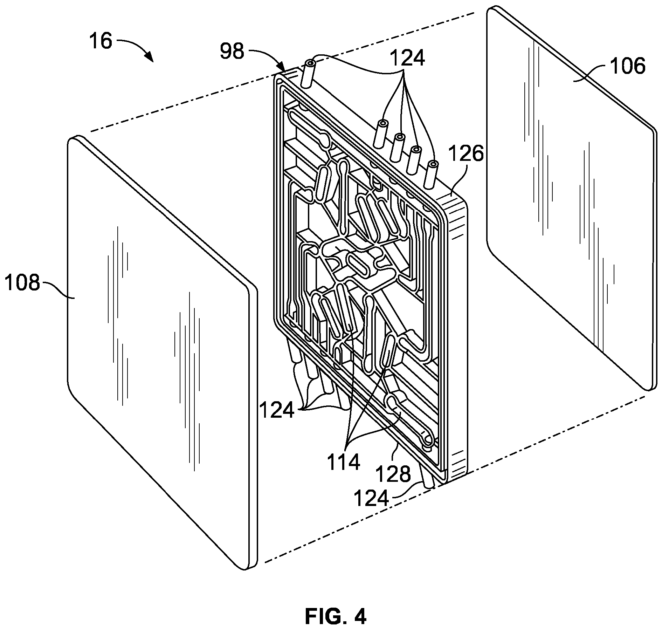

An exemplary cassette 16 is shown in Figs. 3 and 4. In the illustrated example of the flow circuit 12, the cassettes 16-16b may be identically formed, such that the cassette 16 illustrated in Figs. 3 and 4 is representative of cassettes 16a and 16b as well. In one embodiment, the cassette 16 may be of the type shown and described in US Pat. No. 10,413,652 assigned to FENWAL, INC. and incorporated herein by reference in its entirety.

With reference to Figs. 3 and 4, each cassette 16-16b includes a cassette body 98 having a plurality of preformed cavities forming an array of valve stations 110 and an array of pressure sensing stations 112 on one side of the body 98 (Fig. 3) and an array of channels or paths 114 for conveying liquids on the other side (Fig. 4). The valve stations 110 communicate with the liquid paths 114 through an interior wall 100 to interconnect them in a predetermined manner. The sensing stations 112 also communicate with the liquid paths 114 through the interior wall 100 to sense pressures in selected regions. The number and arrangement of the valve stations 110, pressure sensing stations 112 and liquid paths 114 may vary but, in the illustrated embodiment, the cassette 16 provides ten valve stations 110, four sensing stations 112 and nineteen liquid paths 114.

The valve and sensing stations 110, 112 resemble shallow wells open on one side of the cassette 16 surrounded by upstanding edges 116. The valve and sensing stations 110, 112 are closed by the interior wall except that the valve stations 110 include a pair of through holes or ports 118 in the interior wall 100 and the sensing stations 112 include three through holes or ports 120 in the interior wall 100. The ports 118 each open into selected different liquid paths 114 and the ports 120 open into selected liquid paths 114. The ports 120 channel liquid flow among the selected liquid paths 114 through the associated sensing station.

A flexible diaphragm 106 may overlie the one side of the cassette 16 (i.e., the side having the open valve and sensing stations 110, 112) and a rigid panel 108 may overlie the other side of the cassette 16 (i.e., the side having the liquids paths 114). The cassette 16 may also include a plurality of preformed tube connectors 124 extending from side edges 126, 128 of the cassette 16. In the illustrated examples, each cassette 16-16b includes ten pre-molded tube connectors 124, including five extending from one side edge 126 and five extending from an opposite side 128. It will be appreciated that fewer or more preformed tube connectors 124 may be provided on one or both of the sides 126, 128 in a predetermined manner during manufacture of the cassettes 16-16b. The tube connectors 124 communicate with various associated liquid paths 114 such that liquid may enter or exit the liquid paths 114 via the tube connectors 124. Other liquid paths 114 of the cassette may link the associated liquid paths 114 to each other through the valve and sensing stations 110, 112. The tube connectors 124 are configured to be fluidically connected to associated external tubing segments of the flow circuit 12, as shown in Fig. 2.

Referring now to Figs. 1, 5 and 6, the reusable separation device 10 may be of the type shown and described in the aforementioned US Pat. No. 10,413,652, which is incorporated by reference. The reusable separation device 10 includes a plurality of cassette holders 94 arranged on a face 96 of the device 10. The cassette holders 94 are configured to receive respective cassettes 16-16b and grip the respective cassettes 16-16b at opposed side edges. The device 10 further includes a plurality of pump stations 92 configured to interact with the cassettes 16-16b to facilitate fluid flow in the tubing and/or cassettes 16-16b of the flow circuit 12. The cassette holders 94 are configured to receive and position the cassettes 16-16b such that the pump stations 92 operatively engage corresponding tubing loops 50 extending from the cassettes 16-16b (see Figure 2). In the illustrated example, each cassette holder 94 may be provided with a pair of pump stations 92 configured to interact with corresponding tubing loops 50 on the cassette 16. The pump stations 92 may include peristaltic pumps which operatively engage the tubing loops 50.

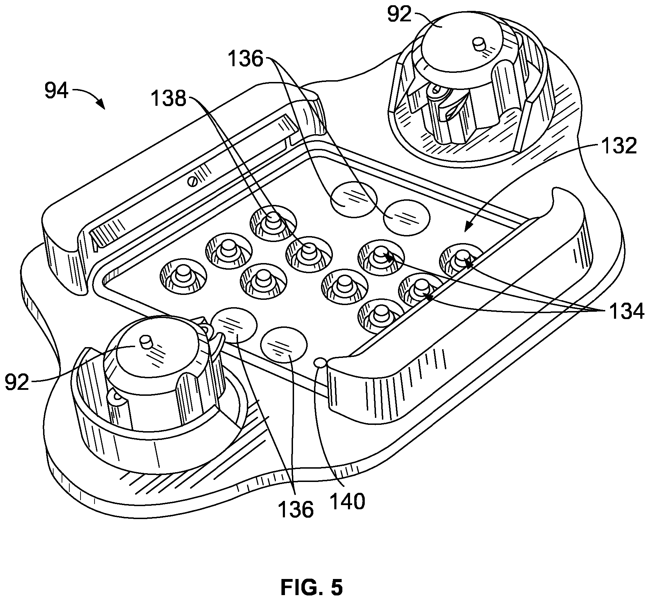

As shown in Fig. 5, the cassette holders 94 each include a valve and sensor array 132 arranged so as to be aligned with corresponding valve and sensing stations 110, 112 of the cassettes 16-16b. In one example, the flexible diaphragm 106 is urged into intimate contact with the valve and sensor array 132 by the cassette holder 94. The valve and sensor array 132 includes a plurality of valve actuators 134 which may correspond in number and/or position to the valve stations 110 of the cassette 16, and a plurality of pressure sensing transducers 136 which may correspond in number and/or position to the sensing stations 112 of the cassette 16.

Each valve actuator 134 may include an electrically actuated solenoid pin or piston 138. The pistons 138 may be independently operated to extend so as to press against the flexible diaphragm 106 in region overlying the associated valve station 110, which closes the associated valve station 110. The pistons 138 may also be independently operated to retract so as to release a force applied against the diaphragm 106. Accordingly, the diaphragm 106 may be unseated from the valve ports 118 of the associated valve station 110, thereby opening the valve station 110 for liquid flow through the ports 118. Alternatively, a vacuum may be applied to the diaphragm 106, for example, by vacuum port 140, to actively unseat the diaphragm from the valve port 118.

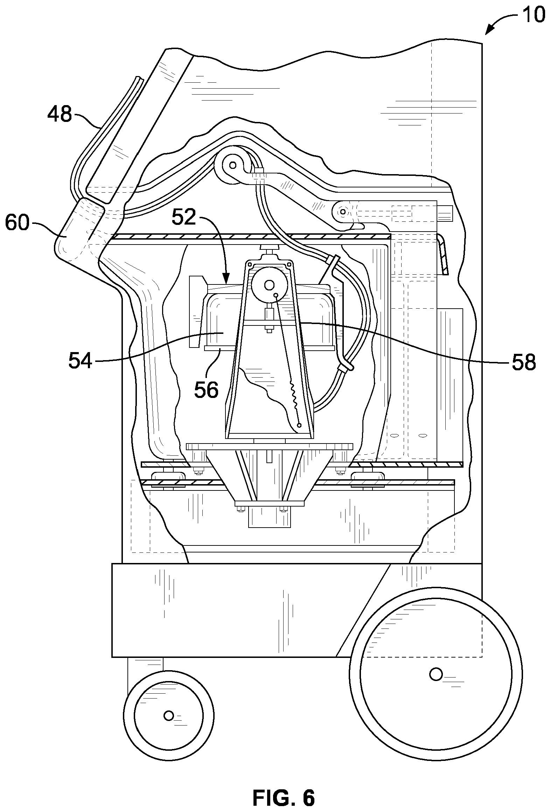

As shown in Fig. 6, the separation device 10 includes a centrifuge 52 used to centrifugally separate blood components. The centrifuge 52 may be programmed to separate blood into a variety of components. For example, the centrifuge 52 may separate whole blood into cellular components (e.g., red blood cells and platelets) and substantially cell-free plasma to perform a TPE procedure according to the present embodiments. The centrifuge 52 may be of the type shown in US Pat. No. 5,316,667 to Brown et al., which is incorporated herein by reference in its entirety. The centrifuge 52 may include a bowl 54 and a spool 56 pivoted on a yoke 58 between an operating position and loading/unloading position. The centrifuge 52 may be housed in the interior of the device 10, and a door 60 may be provided to allow selective access to the centrifuge.

In the loading/unloading position, the spool 56 may be opened and the operator may load the flexible blood separation chamber 34 about the spool 56. Closure of the spool 56 and bowl 54 encloses the chamber 34 for processing. The centrifuge 52 is configured to be rotated at a speed sufficient to separate whole blood in the separation chamber 34 into component parts, such as red blood cells and substantially cell-free plasma for a TPE procedure. Separation and removal of the component blood parts from the separation chamber 34 is further described in US Pat. 10,413,652, incorporated herein by reference.

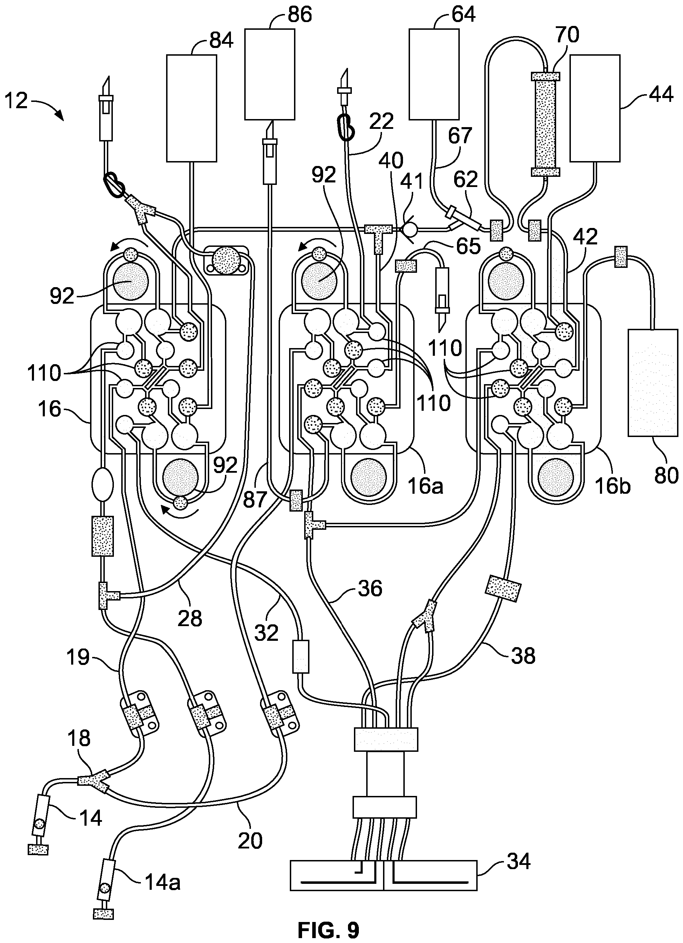

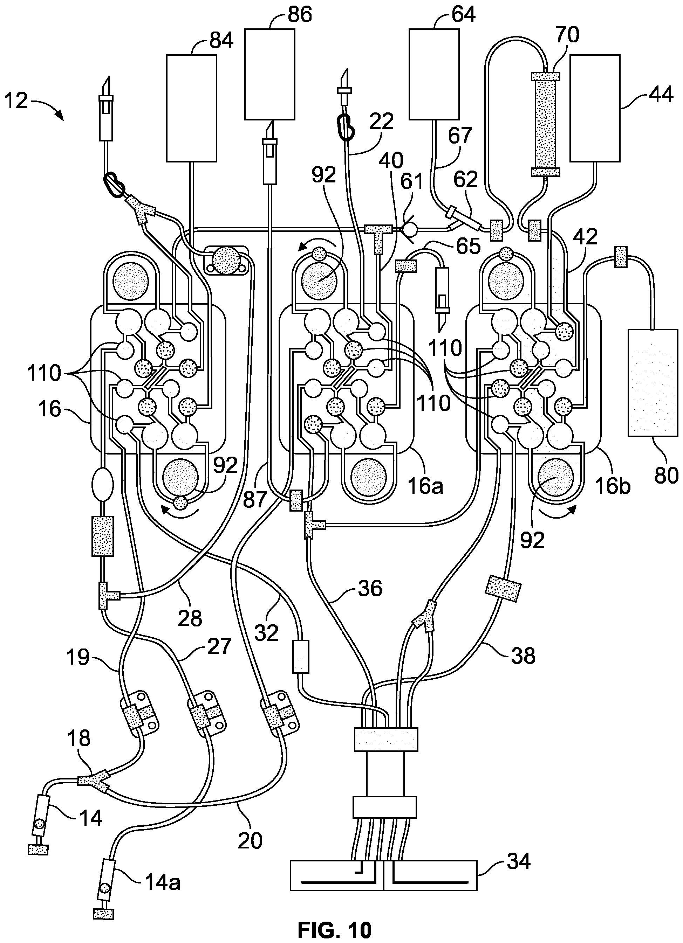

Referring generally to Figs. 1-6, the disposable flow circuit 12 may be arranged on the separation device 10 by positioning the cassettes 16-16b in corresponding cassette holders 94, and further, by arranging the separation chamber 34 in the centrifuge 52, with the umbilicus 48 connecting the separation chamber 34 to the remainder of the flow circuit 12. Another collection container 84 may be fluidically connected to the left cassette 16 and may receive a fluid from the left cassette 16 via tubing 85. A first container 86 (see Figs. 7-10) may be fluidically connected to the middle cassette 16a by tubing 87 and is configured to receive or supply a fluid. In one configuration, a second container 64 (see Figs. 7-10) may be fluidically connected to the middle cassette 16a by tubing 65, and may be configured to receive a fluid from, or supply a fluid to, the middle cassette 16a. The tubing 65 may include a second container connector 66, which may be Luer connection. In another configuration (see Figs. 9 and 10), the second collection container 64 may be fluidically connected to the Luer activated port 62 by tubing 67. In one example, the first and/or second containers 86, 64 may supply a replacement fluid to the middle cassette 16a for a replacement procedure (i.e., to return replacement fluid to the patient instead of plasma).

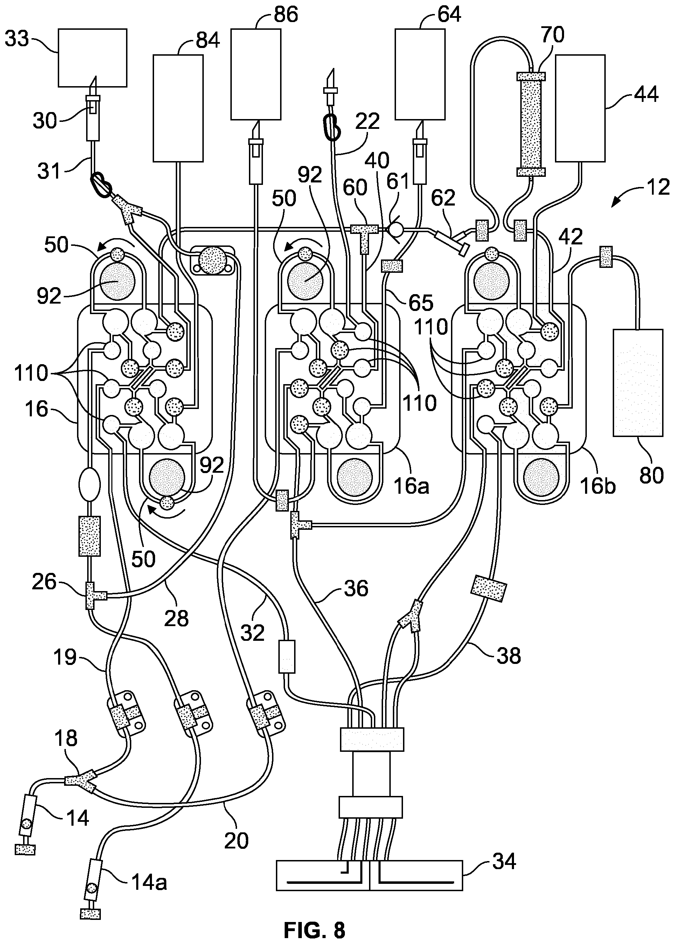

According to aspects of the present disclosure, the flow circuit 12 may be operated in at least four different configurations to carry out selected procedures, by selectively operating one or more of the pump stations 92, one or more of the valve stations 110, and/or selectively connecting/disconnecting portions of the flow circuit 12 to one another and/or connecting/disconnecting additional components to the flow circuit 12 as described further below. Fig. 2 shows an example of the present flow circuit 12 in a first or initial configuration which may be used in a replacement fluid stage of a Replacement Fluid TPE procedure. Fig. 7 shows an example of the present flow circuit 12 in a second configuration for a flush stage of a Columns TPE procedure using a replacement fluid line. Fig. 8 shows an example of the present flow circuit 12 in a third configuration for a divert prime stage of a Columns TPE procedure using the replacement fluid line. Fig. 9 shows an example of the present flow circuit 12 in a fourth configuration for a first step of a divert prime stage for a Columns TPE procedure using a Luer activated port. Fig. 10 shows an example of the present flow circuit 12 in a fifth configuration for a second step of the divert prime stage for a Column TPE procedure using the Luer activated port.

### First Configuration - Replacement Fluid TPE

In the first, or initial configuration shown in Fig. 2, the flow circuit 12 is configured for use with the separation device 10 to perform a replacement fluid stage of a Replacement Fluid TPE procedure. For the replacement fluid stage, one of the pump stations 92 of the left cassette 16 is operated to draw whole blood into the left cassette 16 through blood source access device 14, first y-connector 18 and tubing 19. Anti-coagulant fluid may be received at the first y-connector 18 via an anticoagulant container (not shown), tubing 22 and the middle cassette 16a. The anticoagulant fluid is combined with the whole blood at the first y-connector 18 and flows to the left cassette 16 through tubing 19. In one example, one of the pump stations 92 of the middle cassette 16a may be operated to draw the anti-coagulant fluid from the container, through the middle cassette 16a and to the first y-connector 18. In addition, the valve stations 110 of the middle cassette 16a may be operated to provide a flow path for the anti-coagulant through the middle cassette 16a. The valve stations 110 of the left cassette 16 are operated to provide a flow path through the left cassette 16 that is fluidically connected to tubing 32. Accordingly, the combined whole blood and anti-coagulant may flow out of the left cassette 16 and into the separation chamber 34 via the tubing 32.

The centrifuge 52 is operated at a rotational speed to separate the whole blood in the separation chamber 34 into a cellular component (e.g., red blood cells) and a substantially cell-free plasma component. The cellular component is removed from the separation chamber 34 via tubing 36 and is received in the middle cassette 16a.

The valve stations 110 of the middle cassette 16a may be controlled to provide a flow path through the middle cassette 16a to receive the cellular component via tubing 36. The cellular component may flow out from the middle cassette 16a via tubing 40 and into the left cassette 16, for subsequent return to the blood source via tubing 27 and the other blood source access device 14a. A replacement fluid may be provided to the middle cassette 16a from the first and/or second container 86, 64 attached to tubing 87 or tubing 65 and subsequently directed through the left cassette 16 to the blood source (e.g., the patient) via tubing 27. In one example, the valve stations 110 of the left cassette 16 are controlled to provide a flow path or paths through which the cellular component and the replacement fluid may flow. The other pump station 92 of the left cassette 16 may be operated to draw the cellular component and the replacement fluid into the cassette 16, and further, to promote flow of the cellular component and replacement fluid through tube 27 to return to the blood supply.

The plasma component is removed from the separation chamber 34 and received in the right cassette 16b. In one example, one of the pump stations 92 of the right cassette 16b is operated to draw the plasma component into the right cassette 16b. The valve stations 110 of the right cassette 16b may be operated to provide a flow path through which the plasma component flows for collection in at least one of the collection containers 44 (via tubing 46) and 80 via tubing 81.

Accordingly, in the replacement fluid stage of Replacement Fluid TPE, a volume of replacement fluid may be returned to the blood source via the middle and left cassettes 16a, 16 to replace a volume of substantially cell-free plasma collected in at least one of the containers 44, 80 via the right cassette 16b. Additionally, in the replacement fluid stage, the processes described above may be performed simultaneously. For example, whole blood may be drawn into the left cassette 16 from the blood supply while anticoagulated whole blood is separated in the separation chamber into cellular and substantially cell-free plasma components. The cellular components may be directed through the middle cassette 16a and returned to the blood supply, e.g., a patient, via the left cassette 16, replacement fluid may be directed through the middle cassette 16a and to the blood supply via the left cassette 16, and the substantially cell free plasma may be collected via the right cassette 16b while whole blood is separated in the separation chamber 34. In this manner, return fluid flow (e.g., the cellular components and replacement fluid) to the blood supply, e.g., the patient, may be maintained while whole blood is drawn from the blood supply (e.g., the patient), such that the volume of fluid in the blood supply remains substantially constant or within a predetermined acceptable range.

### Second Configuration - Flush Stage for Columns TPE

In the second configuration, shown in Fig. 7, the adsorption device 70 is connected to the flow circuit 12. In the illustrated example, the adsorption device 70 includes a housing, such as a column, in which the plasma is treated, and second column tubing 73 connected to the column and second column connectors 72. The first and second column tubing 73 include respective parts of the second column connector 72, which may be provided as a Luer connector. The respective parts of the second column connection 72 may be configured to mate with corresponding mating parts of the first column connector 180 so that the corresponding parts of the first and second column connectors 180, 72 may be connected. In this manner, the adsorption device is fluidically connected in line with flow circuit 12.

After connecting the adsorption device 70 to the flow circuit 12, a flush stage may be performed using saline from the saline supply container 33. The flush stage is performed to prepare the adsorption device 70 for use, for example, by flushing residual contents from the column before being fluidically connected to patient.

With further reference to Fig. 7, in the flush stage, saline is drawn from the supply container 33 through tubing 31, the left cassette 16 and into the separation chamber 34 via tubing 32. The valve stations 110 of the left cassette are controlled to provide a flow path for the saline through the cassette 16, and one of the pump stations 92 associated with the left cassette 16 is operated to draw the saline from the container 33, through the left cassette 16 and to the separation chamber 34. Subsequently, the saline may be removed from the separation chamber 34 via tubing 38, through the right cassette 16b and into the adsorption device 70 via tubing 42. The saline further flows from the adsorption device 70 through Luer activated port 62 and check valve 61 into the middle cassette 16a via tubing 40, and from the middle cassette 16a into the first container 86 and/or second container 64 via respective tubings 87, 65. In this example, the first and/or second containers 86, 64 may be considered a first flush fluid collection container 86 and a second flush fluid collection container 64, respectively. In this manner, some volume of saline is transferred through the adsorption device 70 to the flush fluid collection container 86 (or 64) which can be removed or isolated from flow circuit 12.

The flush fluid collection container 86 may be connected to a scale or other load sensor of the device 10 configured to detect a weight of the flush fluid container 86. In this manner, a weight of collected flush fluid, e.g., saline, may be monitored by the device 10 and a volume of the collected flush fluid may be determined.

Further, in the flush stage, the valve stations 110 of the left cassette 16 are operated to block flow paths coming in from tubing 19 and tubing 40, and to block flow paths leading out to left cassette 16 to collection container 84. The valve stations 110 of the right cassette 16b are operated to block flow paths out to the collection containers 44 and 80.

### Third Configuration - Divert Prime Stage for Columns TPE using Replacement Line

In the third configuration of the flow circuit 12, shown in Fig. 8, a divert prime stage may be performed for a Columns TPE procedure. The divert prime stage may follow the flush stage described above. When the divert prime stage begins, saline from the flush stage is still in the adsorption device 70. In the divert prime stage, whole blood is drawn into the flow circuit 12 and anticoagulant from the anticoagulant container flows in the tubing 22, middle cassette 16a and tubing 20 to be combined with whole blood at first y-connector 18 prior to entering the left cassette 16. The anticoagulated whole blood flows from the left cassette 16 into the separation chamber 34, where it may be separated into a cellular component and a substantially cell-free plasma component.

The plasma component flows into the right cassette 16b via tubing 38 and flows out of the right cassette 16b via tubing 42. The plasma component flows into the adsorption device 70 and displaces the saline remaining in the adsorption device 70 from the flush stage. The displaced saline flows through the Luer activated port 62 and check valve 61 into the middle cassette 6a via tubing 40. The saline is directed along a flow path within the middle cassette 16a to the divert prime tubing 65 and into the second container 64, which in this example, may be considered as a divert prime container 64. Thus, in the divert prime stage using the flow circuit 12 in the third configuration, the separated plasma displaces the saline (or other replacement or flush fluid) remaining in adsorption device 70 to be collected in the divert prime container 64. In this manner, a volume of the saline from the flush stage may be removed from the flow circuit 12. Accordingly, a volume of saline used in the flush stage that is subsequently returned to blood source (e.g., a patient) may be reduced or limited.

In the divert prime stage using the third configuration of the flow circuit 12, a sufficient volume of plasma for displacing the saline from the adsorption device 70 is removed from the separation chamber 34 while the cellular component, e.g., red blood cells, may remain in the separation chamber 34.

One of the pump stations 92 associated with the left cassette 16 is operated to draw anticoagulated whole blood into the left cassette 16 and the other pump station 92 associated with the left cassette 16 is operated to send some of the anticoagulated whole blood through 14a to maintain flow to the patient access. The valve stations 110 of the left cassette 16 are operated to provide a first flow path for the whole blood received from tubing 19 within the left cassette 16 to be sent to the separation chamber 34 through tubing 32 and a second flow path for the whole blood to be sent to the patient access through connector 26 and blood supply device 14a. The valve stations 110 are also operated to block flow received from connector 60 through the left cassette 16.

One of the pump stations 92 associated with the middle cassette 16a is operated to draw the anti-coagulant fluid from the anti-coagulant container (not shown) via tubing 22 to the first y-connector 18 where it is combined with the whole blood before flowing into the left cassette 16. The valve stations 110 of the middle cassette 16a are operated to provide a first flow path through which the displaced saline from the adsorption device 70 may flow to the divert prime tubing 65 into the divert prime container 64. The valve stations 110 are also operated to provide a second flow path through which the anti-coagulant fluid may flow through the middle cassette 16a. The valve stations 110 are also operated to block flow through the middle cassette 16a of a fluid received via tubing 36.

The valve stations 110 of the right cassette 16b are operated to provide a flow path through the right cassette 16b to the tubing 42 for the plasma. The valve stations 110 are also operated to block flow out of the right cassette 16b to the collection containers 44, 80.

The divert prime stage using the third configuration of the flow circuit 12 may be suitable when the volume of adsorption device 70 (i.e., volume of column) is relatively low, i.e., about 60 mL or lower.

### Fourth Configuration - Divert Prime Stage using Luer Port (Step 1)

The fourth configuration of the flow circuit 12, shown in Fig. 9, may be used in another divert prime stage for a Columns TPE procedure. The divert prime stage using the fourth configuration may follow the flush stage described above in which the flow circuit 12 is in the second configuration. In this divert prime stage, the divert prime collection container 64 may be fluidically connected to the Luer activated port 62 (via tubing 67) instead of the middle cassette 16a.

Whole blood is drawn into the left cassette 16 and then flows to the separation chamber 34 in the manner described above. The whole blood is separated into a cellular component (e.g., red blood cells) and a substantially cell-free plasma component in the separation chamber 34. The plasma component is removed from the separation chamber 34 and flows through the right cassette 16b to tubing 42, and subsequently into the adsorption device 70. The plasma component displaces the saline remaining in the adsorption device 70 from the flush stage, which then flows through the Luer activated port 62 and into the divert prime collection container 64 via tubing 67. During this step (i.e., a first step) of the divert prime stage, the cellular component may remain in separation chamber 34.

One of the pump stations 92 associated with the left cassette 16 is operated to draw whole blood into the left cassette 16 and the other pump station 92 associated with the left cassette 16 is operated to send some of the anticoagulated whole blood through 14a to maintain flow to the patient access. The valve stations 110 of the left cassette 16 are operated to provide a first flow path for the whole blood received from tubing 19 to be sent to the separation chamber 34 through tubing 32 and a second flow path for whole blood to be sent to the patient access through connector 26 and blood supply device 14a. The valve stations 110 are also operated to block flow from tubing 40 through the left cassette 16 and to block flow from the collection container 64 connected between middle cassette 16a and right cassette 16b.

One of the pump stations 92 associated with the middle cassette 16a is operated to draw the anti-coagulant fluid from the anti-coagulant container (not shown) to the first y-connector 18 where it is combined with the whole blood before flowing into the left cassette 16. The valve stations 110 of the middle cassette 16a are operated to provide a first flow path through which the anti-coagulant fluid may flow to tubing 20. The valve stations 110 are also operated to block flow from tubing 36 through the cassette 16a, and to block flow from the cassette 16a to the flush fluid collection container 86 and to the tubing 65, which is not connected to the divert prime container 64 in this configuration.

The valve stations 110 of the right cassette 16b are operated to provide a flow path through the right cassette 16b to the tubing 42 for the plasma. The valve stations 110 are also operated to block flow out of the right cassette 16b to the collection containers 44, 80.

The divert prime stage using the fourth configuration of the flow circuit 12 may be suitable when the volume of adsorption device 70 (i.e., volume of column) is relatively high, for example, 150 mL or more. The fourth configuration may be suitable as a first step of a two step divert prime stage as well. In some examples, to accommodate a larger volume of replacement fluid displaced from the adsorption device 70, the divert prime collection container 64 may be replaced with, for example, a larger capacity collection container, such as one of the collection containers 80.

### Fifth Configuration - Divert Prime Stage using Luer Port (Step 2)

The fifth configuration of the flow circuit 12, shown in Fig. 10, may be used in a second step of a divert prime stage for a Columns TPE procedure. The second step of the divert prime stage using the fifth configuration may follow the first step of the divert prime stage described above in which the flow circuit 12 is in the fourth configuration.

In this second step, the cellular components may be removed from the separation chamber 34 through tubing 36. The valve stations 110 of the middle cassette 16a are controlled to provide a second flow path through the middle cassette 16a for the cellular components received from the tubing 36. The cellular component may flow out of the middle cassette 16a through tubing 40 to be received in the left cassette 16. The valve stations 110 of the left cassette 16 are controlled to provide a flow path for cellular components to flow to the tubing 27 for return to the blood supply via blood supply device 14a. The check valve 61 prevents backflow of the cellular component from the left cassette 16 and/or the middle cassette 16a to the adsorption device 70.

The substantially cell-free plasma component may continue to be removed from the separation chamber 34 and flow through the right cassette 16b into the adsorption device 70. The plasma may then continue through the Luer activated port and into the divert prime collection container 64.

One of the pump stations 92 associated with the left cassette 16 is operated to change to an off condition and disengaged to allow flow through the pump tubing in step 2 of this prime divert stage from an on condition in step 1 of the prime divert stage. In addition, one of the pump stations 92 associated with the right cassette 16b may be operated to continue to draw the plasma component from the separation chamber 34 into the right cassette 16b.

### Controller

The device 10 further includes a controller operably coupled to the valve and sensing array 132 to control operation of the valve actuators. For example, the controller may operate a valve actuator to retract and thereby open a corresponding valve station 110 of a cassette 16-16b. Alternatively, or in addition, the controller may operate a valve actuator to extend and thereby close a corresponding valve station 110. It will be appreciated that in some examples, the valve actuators may be urged to either an open or closed position, and the valve actuator may be controlled to move in a direction against a biasing force urging the actuator to the open or closed position.

In addition, the controller may be operably connected to one or more scales which interact with a container to detect a weight of the container. The controller may receive information indicative of the detected container weight and determine the weight based on the received information indicative of the detected container weight. In this manner, the controller may be configured to monitor a weight of, for example, a container configured to collect saline (or other flush fluid) used in the flush stage of a Columns TPE procedure, a container configured to collect fluid in a divert prime stage, and/or a container configured to collect plasma in a Replacement TPE procedure. In one embodiment, the flush collection container 86 is operably connected to a first scale, the divert prime container 64 is operably connected to a second scale, and the collection container 44 is operably connected to a third scale.

The controller may also be operably connected to the pump stations 92 and may control the pumps stations 92 to start/stop, operate at a selected or programmed speed or speeds, and/or at selected or programmed time intervals. In one embodiment, the controller may be preprogrammed to operate the valve actuators and the pump stations in a predetermined manner to perform one or more the TPE procedures or stages of the TPE procedures described herein, and/or one or more other blood treatment procedures.

In some examples, the controller is configured to control operations of the pump stations and valve actuators to perform the Replacement TPE procedure and Column TPE procedures described herein, and further, to automatically transition from one stage to another (i.e., to end one stage and begin another). In one example, when a predetermined volume of saline is collected by the divert prime container 64, as determined based on the detected weight of the container 64, the controller may automatically stop diversion and alert the operator to remove or change the container 64. Alternatively, the operator may have the option to end diversion, remove and replace the container 64, and then resume the procedure. That is, a volume of saline delivered to the divert prime container 64 may be monitored by the controller and used to determine completion of a step (e.g., step 1 of the divert prime stage) and, in some examples, begin step 2 of the divert prime stage by operating the pump and valve stations to configure the flow circuit in the fifth configuration.

In some examples, once the adsorption device 70 is filled with plasma, the controller may automatically stop diversion and alert the operator to remove the divert prime container 64.

The controller may be implemented, for example, as a computing device or the like having a processor, such as a microprocessor, and a memory operably coupled to the processor, wherein the memory is configured to store program instructions which are executable by the processor to perform one or more functions, such as a function to control or operate a component of the device, such as a pump station or valve actuator. The controller may also be configured to receive, store and process information received from other components, such as sensors or data input devices. The controller may also transmit information, including signals, instructions and/or data to other electronic devices.

Other suitable configurations of the flow circuit are also possible even if not depicted in the present figures. For example, it is envisioned that the third configuration (Fig. 8) may be modified or reconfigured for use with a relatively large volume adsorption container 70. As one example, it is envisioned that the divert prime container 64 may be disconnected from tubing 65 at connector 66 and the larger volume collection container 80 may be disconnected from tubing 81 at connector 82. The larger volume collection container 80 may then be connected to tubing 65 (which is fluidically connected to the middle cassette 16a) by connecting the connectors 66 and 82.

### Aspects

Aspect 1. A blood treatment system comprising a reusable separation device comprising a plurality of cassette holders and a centrifuge; and a flow circuit comprising a plurality of cassettes, each cassette configured to be arranged on a corresponding cassette holder, a separation chamber fluidically connected to the plurality of cassettes and configured to be arranged on the centrifuge, and a plurality of tubings fluidically connecting the plurality of cassettes and the separation chamber, the plurality of tubings including a plasma tubing including a first column connector, a Luer activated port, and a check valve, wherein the flow circuit has a first configuration for a replacement fluid therapeutic plasma exchange procedure in which the first column connector has first and second mating parts connected to one another, and a second configuration for an adsorption therapeutic plasma exchange procedure in which the flow circuit further comprises an adsorption device having a second column connector and the adsorption device is connected in line with plasma tubing and the first and second mating parts of the first column connector are connected to corresponding first and second parts of the second column connector.

Aspect 2. The blood treatment system of Aspect 1, wherein each of the cassette holders includes a valve and sensor array having a plurality of valve actuators and further includes two associated pump stations and each of the cassettes includes a plurality of valve stations and sensor stations on one side, and a plurality of fluid paths on another side, wherein the valve actuators are selectively operable to actuate corresponding valve stations to provide different flow paths on the cassettes formed by one or more of the fluid paths, and wherein the pump stations are selectively operable to cause fluid flow through the flow paths.

Aspect 3. The blood treatment system of Aspect 2, wherein the plurality of cassettes includes a left cassette, a middle cassette and a right cassette, wherein: the left cassette is configured to receive whole blood from a blood supply, the separation chamber is configured to receive the whole blood from the left cassette, the centrifuge is configured to be operated to separate the whole blood in the separation chamber into a cellular component and a substantially cell-free plasma component, the middle cassette is configured to receive the cellular component from the separation chamber, the right cassette is configured to receive the plasma from the separation chamber, and the plasma tubing is fluidically connected to and extends downstream from the right cassette.

Aspect 4. The blood treatment system according to Aspect 3, wherein in the first configuration: a replacement fluid container having a replacement fluid is fluidically connected to a middle cassette and the valve stations and pump stations are operated to direct the replacement fluid through the middle cassette to the blood supply, a collection container is fluidically connected to the right cassette and the valve stations and pump stations are operated to direct the plasma through the right cassette to the collection container.

Aspect 5. The blood treatment system according to Aspect 4, wherein in the second configuration: a flush fluid is fluidically connected to the left cassette, a flush fluid collection container is fluidically connected to the middle cassette, and the valve stations and pump stations are operated to direct the flush fluid through the left cassette, the separation chamber, the right cassette, the adsorption device, the Luer activated port, the check valve and the middle cassette to the flush fluid collection container.

Aspect 6. The blood treatment system according to Aspect 5, wherein the flow circuit has a third configuration in which: a divert prime collection container is fluidically connected to the middle cassette, the valve stations and pump stations are operated to direct the whole blood to the separation chamber, the whole blood is separated into the cellular component and substantially cell-free plasma in the separation chamber, and the valve stations and pump stations are further operated to remove the plasma from the separation chamber, through the right cassette and into the adsorption device, such that the plasma displaces the flush fluid from the adsorption device into the middle cassette and from the middle cassette into the divert prime collection container.

Aspect 7. The blood treatment system according to Aspect 6, wherein a volume of the adsorption device is 60 mL or less.

Aspect 8. The blood treatment system according to Aspect 5, wherein the flow circuit has a fourth configuration in which: a divert prime collection container is fluidically connected to the Luer activated port, the valve stations and pump stations are operated to direct the whole blood into the left cassette and to the separation chamber, the whole blood is separated into the cellular component and substantially cell-free plasma in the separation chamber, and the valve stations and pump stations are further operated to remove the plasma from the separation chamber, through the right cassette and into the adsorption device, such that the plasma displaces the flush fluid from the adsorption device through the Luer activated port and into the divert prime collection container.

Aspect 9. The blood treatment system according to Aspect 8, wherein the flow circuit has a fifth configuration in which: the valve stations and the pump stations are operated to provide the cellular component from the separation chamber into the middle cassette, from the middle cassette to the left cassette via corresponding tubing, and from the left cassette to the blood supply, wherein the check valve prevents back flow of the cellular material toward the adsorption device, while the valve stations and the pump stations continue to provide the plasma to the adsorption device.

Aspect 10. The blood treatment system according to Aspect 9, wherein the divert prime collection container is connected to a scale, and the valve stations and pump stations are operated to change the flow circuit from the fourth configuration to the fifth configuration in response to the divert prime collection container reaching a predetermined weight.

Aspect 11. The blood treatment system according to any one of Aspects 8-10, wherein a volume of the adsorption device is 150 mL or more.

Aspect 12. A flow circuit for a blood treatment system, the flow circuit comprising: a plurality of cassettes; a separation chamber fluidically connected to the plurality of cassettes; and a plurality of tubings fluidically connecting the plurality of cassettes and the separation chamber, the plurality of tubings including a plasma tubing including a first column connector, a Luer activated port, and a check valve, wherein the flow circuit has a first configuration for a replacement fluid therapeutic plasma exchange procedure in which the first column connector has first and second mating parts connected to one another, and a second configuration for an adsorption therapeutic plasma exchange procedure in which the flow circuit further comprises an adsorption device having a second column connector and the adsorption device is connected in line with plasma tubing and the first and second mating parts of the first column connector are connected to corresponding first and second parts of the second column connector.

Aspect 13. The flow circuit according to Aspect 12, wherein the plurality of cassettes includes a left cassette, a middle cassette and a right cassette, wherein: the left cassette is configured to receive whole blood from a blood supply, the separation chamber is configured to receive the whole blood from the left cassette, wherein the whole blood is separated into a cellular component and a substantially cell-free plasma component, the middle cassette is configured to receive the cellular component from the separation chamber, the right cassette is configured to receive the plasma from the separation chamber, and the plasma tubing is fluidically connected to and extends downstream from the right cassette.

Aspect 14. The flow circuit according to Aspect 13, wherein in the first configuration, a replacement fluid container having a replacement fluid is fluidically connected to the middle cassette and flow path for the replacement fluid is provided through the middle cassette and the left cassette to the blood supply, and a collection container is fluidically connected to the right cassette such that the plasma is directed through the right cassette to the collection container, wherein flow of the replacement fluid and cellular component to the blood supply via the left cassette and flow of the substantially cell free plasma into the collection container via the right cassette are maintained while whole blood is drawn from the blood supply and received by the left cassette.

Aspect 15. The flow circuit according to Aspect 14, wherein in the second configuration: a flush fluid is fluidically connected to the left cassette, a flush fluid collection container is fluidically connected to the middle cassette, and a flow path for the flush fluid is provided through the left cassette, the separation chamber, the right cassette, the adsorption device, the Luer activated port, the check valve and the middle cassette to the flush fluid collection container.

Aspect 16. The flow circuit according to Aspect 15, wherein the flow circuit has a third configuration in which: a divert prime collection container is fluidically connected to the middle cassette, a flow path for the whole blood is provided into the left cassette and to the separation chamber, the whole blood is separated into the cellular component and substantially cell-free plasma in the separation chamber, and a flow path is provided to remove the plasma from the separation chamber, through the right cassette and into the adsorption device, such that the plasma displaces flush fluid from the adsorption device into the middle cassette and from the middle cassette into the divert prime collection container.

Aspect 17. The flow circuit according to Aspect 16, wherein a volume of the adsorption device is 60 mL or less.

Aspect 18. The flow circuit according to Aspect 15, wherein the flow circuit has a fourth configuration in which: a divert prime collection container is fluidically connected to the Luer activated port, a flow path is provided for the whole blood into the left cassette and to the separation chamber, the whole blood is separated into the cellular component and substantially cell-free plasma in the separation chamber, and a flow path is provided to remove the plasma from the separation chamber, through the right cassette and into the adsorption device, such that the plasma displaces flush fluid from the adsorption device through the Luer activated port and into the divert prime collection container.

Aspect 19. The flow circuit according to Aspect 18, wherein the flow circuit has a fifth configuration in which: a flow path is provided for the cellular component from the separation chamber into the middle cassette, from the middle cassette to the left cassette via corresponding tubing, and from the left cassette to the blood supply, wherein the check valve prevents back flow of the cellular material toward the adsorption device, and a flow path further provides the plasma to the adsorption device.

Aspect 20. The flow circuit according to Aspect 19, wherein the divert prime collection container is connected to a scale, and the flow circuit is changed from the fourth configuration to the fifth configuration in response to the divert prime collection container reaching a predetermined weight.

Aspect 21. The flow circuit according to any one of Aspects 18-20, wherein a volume of the adsorption device is 150 mL or more.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A blood treatment system comprising:
a reusable separation device comprising a plurality of cassette holders and a centrifuge; and
a flow circuit comprising a plurality of cassettes, each cassette configured to be arranged on a corresponding cassette holder, a separation chamber fluidically connected to the plurality of cassettes and configured to be arranged on the centrifuge, and a plurality of tubings fluidically connecting the plurality of cassettes and the separation chamber, the plurality of tubings including a plasma tubing including a first column connector, a Luer activated port, and a check valve,
wherein the flow circuit has a first configuration for a replacement fluid therapeutic plasma exchange procedure in which the first column connector has first and second mating parts connected to one another, and a second configuration for an adsorption therapeutic plasma exchange procedure in which the flow circuit further comprises an adsorption device having a second column connector and the adsorption device is connected in line with plasma tubing and the first and second mating parts of the first column connector are connected to corresponding first and second parts of the second column connector.

2. The blood treatment system of claim 1, wherein each of the cassette holders includes a valve and sensor array having a plurality of valve actuators and further includes two associated pump stations,
wherein each of the cassettes includes a plurality of valve stations and sensor stations on one side, and a plurality of fluid paths on another side,
wherein the valve actuators are selectively operable to actuate corresponding valve stations to provide different flow paths on the cassettes formed by one or more of the fluid paths, and
wherein the pump stations are selectively operable to cause fluid flow through the flow paths.

3. The blood treatment system of claim 2, wherein the plurality of cassettes includes a left cassette, a middle cassette and a right cassette, wherein:
the left cassette is configured to receive whole blood from a blood supply,
the separation chamber is configured to receive the whole blood from the left cassette,
the centrifuge is configured to be operated to separate the whole blood in the separation chamber into a cellular component and a substantially cell-free plasma component,
the middle cassette is configured to receive the cellular component from the separation chamber,
the right cassette is configured to receive the plasma from the separation chamber, and
the plasma tubing is fluidically connected to and extends downstream from the right cassette.

4. The blood treatment system according to claim 3, wherein in the first configuration:
a replacement fluid container having a replacement fluid is fluidically connected to the middle cassette and the valve stations and pump stations are operated to direct the replacement fluid through the middle cassette to the blood supply,
a collection container is fluidically connected to the right cassette and the valve stations and pump stations are operated to direct the plasma through the right cassette to the collection container.

5. The blood treatment system according to claim 4, wherein in the second configuration:
a flush fluid is fluidically connected to the left cassette
and
a flush fluid collection container is fluidically connected to the middle cassette, and
the valve stations and pump stations are operated to direct the flush fluid through the left cassette, the separation chamber, the right cassette, the adsorption device, the Luer activated port, the check valve and the middle cassette to the flush fluid collection container.

6. The blood treatment system according to claim 5, wherein the flow circuit has a third configuration in which:
a divert prime collection container is fluidically connected to the middle cassette,
the valve stations and pump stations are operated to direct the whole blood into the left cassette and to the separation chamber,
the whole blood is separated into the cellular component and substantially cell-free plasma in the separation chamber, and
the valve stations and pump stations are further operated to remove the plasma from the separation chamber, through the right cassette and into the adsorption device, such that the plasma displaces the flush fluid from the adsorption device into the middle cassette and from the middle cassette into the divert prime collection container.

7. The blood treatment system according to claim 5, wherein the flow circuit has a fourth configuration in which:
a divert prime collection container is fluidically connected to the Luer activated port,
the valve stations and pump stations are operated to direct the whole blood into the left cassette and to the separation chamber,
the whole blood is separated into the cellular component and substantially cell-free plasma in the separation chamber, and
the valve stations and pump stations are further operated to remove the plasma from the separation chamber, through the right cassette and into the adsorption device, such that the plasma displaces the flush fluid from the adsorption device through the Luer activated port and into the divert prime collection container.

8. The blood treatment system according to claim 7, wherein the flow circuit has a fifth configuration in which:
the valve stations and the pump stations are operated to provide the cellular component from the separation chamber into the middle cassette, from the middle cassette to the left cassette via corresponding tubing, and from the left cassette to the blood supply, wherein the check valve prevents back flow of the cellular material toward the adsorption device while the valve stations and the pump stations continue to provide the plasma to the adsorption device.

9. A flow circuit for a blood treatment system, the flow circuit comprising:
a plurality of cassettes;
a separation chamber fluidically connected to the plurality of cassettes; and
a plurality of tubings fluidically connecting the plurality of cassettes and the separation chamber, the plurality of tubings including a plasma tubing including a first column connector, a Luer activated port, and a check valve,
wherein the flow circuit has a first configuration for a replacement fluid therapeutic plasma exchange procedure in which the first column connector has first and second mating parts connected to one another, and a second configuration for an adsorption therapeutic plasma exchange procedure in which the flow circuit further comprises an adsorption device having a second column connector and the adsorption device is connected in line with plasma tubing and between the first and second mating parts of the first column connector such that the first and second mating parts of the first column connector are connected to corresponding first and second parts of the second column connector.

10. The flow circuit according to claim 9, wherein the plurality of cassettes includes a left cassette, a middle cassette and a right cassette, wherein:
the left cassette is configured to receive whole blood from a blood supply,
the separation chamber is configured to receive the whole blood from the left cassette, wherein the whole blood is separated into a cellular component and a substantially cell-free plasma component,
the middle cassette is configured to receive the cellular component from the separation chamber,
the right cassette is configured to receive the plasma from the separation chamber, and
the plasma tubing is fluidically connected to and extends downstream from the right cassette.

11. The flow circuit according to claim 10, wherein in the first configuration:
a replacement fluid container having a replacement fluid is fluidically connected to the middle cassette, and a flow path for the replacement fluid is provided through the middle cassette and the left cassette to the blood supply, and
a collection container is fluidically connected to the right cassette such that the plasma is directed through the right cassette to the collection container,
wherein flow of the replacement fluid and cellular component to the blood supply via the left cassette and flow of the substantially cell free plasma into the collection container via the right cassette are maintained while the whole blood is drawn from the blood supply and received by the left cassette.

12. The flow circuit according to claim 11, wherein in the second configuration:
a flush fluid is fluidically connected to the left cassette;
a flush fluid collection container is fluidically connected to the middle cassette, and
a flow path for the flush fluid is provided through the left cassette, the separation chamber, the right cassette, the adsorption device, the Luer activated port, the check valve and the middle cassette to the flush fluid collection container.

13. The flow circuit according to claim 12, wherein the flow circuit has a third configuration in which:
a divert prime collection container is fluidically connected to the middle cassette,
a flow path for the whole blood is provided into the left cassette and to the separation chamber,
the whole blood is separated into the cellular component and substantially cell-free plasma in the separation chamber, and
a flow path is provided to remove the plasma from the separation chamber, through the right cassette and into the adsorption device, such that the plasma displaces flush fluid from the adsorption device into the middle cassette and from the middle cassette into the divert prime collection container.

14. The flow circuit according to claim 12, wherein the flow circuit has a fourth configuration in which:
a divert prime collection container is fluidically connected to the Luer activated port,
a flow path is provided for the whole blood into the left cassette and to the separation chamber,
the whole blood is separated into the cellular component and substantially cell-free plasma in the separation chamber, and
a flow path is provided to remove the plasma from the separation chamber, through the right cassette and into the adsorption device, such that the plasma displaces the flush fluid from the adsorption device through the Luer activated port and into the divert prime collection container.

15. The flow circuit according to claim 14, wherein the flow circuit has a fifth configuration in which:
a flow path is provided for the cellular component from the separation chamber into the middle cassette, from the middle cassette to the left cassette via corresponding tubing, and from the left cassette to the blood supply, wherein the check valve prevents back flow of the cellular material toward the adsorption device, and
a flow path further provides the plasma to the adsorption device.
